# EUROPEAN PATENT SPECIFICATION

(11) **EP 3 865 571 B1**
(45) Date of publication and mention of the grant of the patent: **25.12.2024**
(21) Application number: 19867525.8
(22) Date of filing: 27.09.2019
(51) Int. Cl.: C12N 5/0775, A61K 35/28, A61K 35/24, A61P 19/02

(54) **SYNOVIUM-DERIVED MESENCHYMAL STEM CELLS AND USE THEREOF**
AUS SYNOVIUM GEWONNENE MESENCHYMALE STAMMZELLEN UND VERWENDUNG DAVON
CELLULES SOUCHES MÉSENCHYMATEUSES DÉRIVÉES DE LA SYNOVIALE ET LEUR UTILISATION

(30) Priority: 28.09.2018 KR 20180116496
(43) Date of publication of application: 18.08.2021
(73) Proprietor: Hierabio Inc., Seodaemun-gu, Seoul, 03760 (KR)
(72) Inventor: LEE, Seung Jin, Seoul 07333 (KR); KIM, Heejung, Seongnam-si, Gyeonggi-do 13503 (KR); YANG, Young-Il, Busan 48091 (KR)
(74) Representative: Weickmann & Weickmann PartmbB
(86) International application number: PCT/KR2019/012602
(87) International publication number: WO 2020/067774

(56) References cited:
- EP-A1- 3 028 722
- WO-A1-2008/023829
- WO-A1-2016/047849
- KR-A- 20080 068 391
- KR-A- 20130 124 074
- US-A1- 2013 123 939
- TIAGO FERRO ET AL: "Successful isolation and ex vivo expansion of human mesenchymal stem/stromal cells obtained from different synovial tissue-derived (biopsy) samples", JOURNAL OF CELLULAR PHYSIOLOGY, WILEY SUBSCRIPTION SERVICES, INC, US, vol. 234, no. 4, 26 August 2018 (2018-08-26), pages 3973 - 3984, XP071324007, ISSN: 0021-9541, DOI: 10.1002/JCP.27202
- FAN J ET AL: "In vitro engineered cartilage using synovium-derived mesenchymal stem cells with injectable gellan hydrogels", ACTA BIOMATERIALIA, ELSEVIER, AMSTERDAM, NL, vol. 6, no. 3, 1 March 2010 (2010-03-01), pages 1178 - 1185, XP026879163, ISSN: 1742-7061, [retrieved on 20090904]
- BITA CARRION ET AL: "A Safe and Efficient Method to Retrieve Mesenchymal Stem Cells from Three-Dimensional Fibrin Gels", TISSUE ENGINEERING. PART C, METHODS DEC 2008, vol. 20, no. 3, 1 March 2014 (2014-03-01), US, pages 252 - 263, XP055716195, ISSN: 1937-3384, DOI: 10.1089/ten.tec.2013.0051
- JACLYN LOCK ET AL: "Nanophase hydroxyapatite and poly(lactide--glycolide) composites promote human mesenchymal stem cell adhesion and osteogenic differentiation in vitro", JOURNAL OF MATERIALS SCIENCE: MATERIALS IN MEDICINE, KLUWER ACADEMIC PUBLISHERS, BO, vol. 23, no. 10, 7 July 2012 (2012-07-07), pages 2543 - 2552, XP035121197, ISSN: 1573-4838, DOI: 10.1007/S10856-012-4709-0
- PARK SR , KIM SJ: "Treatment of Articular Cartilage Injury Using Mesenchymal Stem Cells", THE JOURNAL OF KOREAN ORTHOPAEDIC RESEARCH SOCIETY, vol. 18, no. 1, 1 June 2015 (2015-06-01), KR, pages 18 - 24, XP009527528, ISSN: 1226-8933, DOI: 10.0000/jkors.2015.18.1.18

## Description

### BACKGROUND OF THE INVENTION

### 1. Field of the Invention

The present invention relates to a method of preparing synovium-derived mesenchymal stem cells using a synovial tissue and a hydrogel, and to a pharmaceutical composition for treating bone or cartilage damage, the pharmaceutical composition including the synovium-derived mesenchymal stem cells.

### 2. Description of the Related Art

Bones support the soft tissues of the body and body weight, and protect the internal organs from external impact by enclosing them. Moreover, bones are one of the important parts of the body that not only structurally support muscles or organs, but also store substances such as calcium or other essential minerals, i.e., phosphorus or magnesium, in the body.

Joints exist between bones that constitute the body. Joints can be classified into immovable joints with little or no mobility between two bones or cartilages that are in contact with each other, such as skull or dental root, movable joints with many connective tissues and high mobility between two bones such as an animal's limb bones or jawbone, and slightly movable, amphiarthrodial joints. Joints generally refer to the movable joints, and the movable joints are classified into ligamentous joints, whereby both bones are connected only with ligaments, and synovial joints. The synovial joint refers to a joint surrounded by a connective tissue capsule (articular capsule). The inside of the articular capsule secretes a synovial fluid having lubricating property, and many ligaments are located outside the articular capsule to strengthen the joint.

Stem cells, which are the cells in the pre-differentiation stage before being differentiated into each cell constituting a tissue, refer to cells having an unlimited proliferation potential in the undifferentiated state, and having a potential for differentiation into cells of various tissues by a specific differentiation stimulus.

Stem cells are largely divided into embryonic stem cells (ES cells) and adult stem cells (tissue-specific stem cells) according to their differentiation potential. Embryonic stem cells are stem cells isolated from inner cell mass (ICM), which is supposed to develop into a fetus, in the very early stage blastocyst before the fertilized egg is implanted in the endometrium. These embryonic stem cells are pluripotent cells that are able to differentiate into every tissue.

In contrast, tissue-specific stem cells are stem cells specific to each organ, which appear at the organ forming stage during embryonic development. Differentiation potential thereof is generally limited to tissue forming cells (multipotent). The representative tissue-specific stem cells are hematopoietic stem cells that exist in bone marrow and mesenchymal stem cells that differentiate into connective tissue cells except blood cells. Hematopoietic stem cells differentiate into various blood cells such as erythrocytes, leucocytes, etc., and mesenchymal stem cells differentiate into osteoblasts, chondroblasts, adipocytes, myoblasts, etc.

Recently, human embryonic stem cells were successfully isolated and the clinical application thereof has been a major concern. The best interest of stem cell application is to use stem cells as a cell supplier for cell replacement therapy.

In the differentiation of mesenchymal stem cells into chondrogenic cells, and further into chondrocytes, that is, in the chondrogenic differentiation, cytokines and growth factors are involved. The exact mechanisms have not been identified, but transforming growth factor beta (TGF-β), insulin-like growth factor (IGF), bone morphogenic protein (BMP), fibroblast growth factor (FGF), etc., is known to play an important role in differentiation into chondrocytes. Accordingly, mesenchymal stem cells are studied to use their ability to differentiate for regeneration of damaged joint tissue, treatment of inflammation, or the like (US Patent No. 6835377). Also, bone marrow-derived stem cells (Majumdar M. K. et al., J. Cell. Physiol. 185: 98-106, 2000), cord blood(Gang E. J. et al., Biochem. Biophys. Res. Commun. 321: 102-108, 2004), and synovium (Fickert S. et al., Osteoarthritis Cartilage 11: 790-800, 2003) are studied as available cell source for treatment of damaged cartilage in addition to the patient's autologous chondrocytes. However, the treatment of bone disease or inflammation using stem cells is not so effective at present. Especially, the treatment of bone disease or inflammation using cartilage stem cells has never been reported.

European patent application EP 3 028 722 A1 discloses synovial MSCs for tissue repair. Further described is the storage of synovial MSCs in hyaluronic acid.

International patent application WO 2008/023829 discloses culturing ex vivo autologous synovium-derived mesenchymal stem cells and implanting the MSCs such that a cartilage defect site is covered by the MSCs and regeneration can occur.

Ferro et al., J. Cell. Physiol., Vol.234, no.4, 2018, p.3973-3984, describes the isolation and cultivation of synovial-derived MSCs that can be used in tissue engineering and cartilage repair.

Fan et al., Acta Biomaterialia, Vol.6, no.3, 2010, p.1178-1186 disclose synovium-derived MSCs isolated from rabbit synovial tissue, amplified to passage 4 in monolayer, and encapsulated in injectable gellan hydrogels. Injectable gels, when treated with TGF-beta1, TGF-beta3 or BMP-2 are deemed competent for in vitro engineered cartilage formation.

Carrion et al., Tissue Engineering Part C, Methods Dec 2008, Vol.20. no.3, 2014, p.252-263, describe a method to recover cells encapsulated within 3D fibrin hydrogelsbased on nattokinase. The method uses mesenchymal stem cell and not synovial tissue.

In view of this technical background, the present inventors have conducted various studies to develop new stem cells that may be used for the treatment of cartilage damage, and as a result, they confirmed that synovial tissue-derived mesenchymal stem cells may effectively treat cartilage damage, thereby completing the present invention.

### SUMMARY OF THE INVENTION

The invention is defined by the appended claims. Additional aspects are provided for illustrative purposes only. It is understood that any references to methods of treatment are to be interpreted as references to the compounds, pharmaceutical compositions and medicaments for use in such methods.

An object of the present invention is to provide a method of preparing synovium-derived mesenchymal stem cells using a synovial tissue and a hydrogel as defined in claims 1 to 3.

The present document also describes synovium-derived mesenchymal stem cells prepared by the above method.

Another object of the present invention is to provide a pharmaceutical composition for treating bone or cartilage damage, the pharmaceutical composition including the synovium-derived mesenchymal stem cells as defined in claims 4 to 8.

The present document also describes a composition for culturing mesenchymal stem cells, the composition including a synovial tissue and a hydrogel.

Still another aspect of the present disclosure is a kit for culturing mesenchymal stem cells, the kit including the composition.

Still another aspect of the present disclosure is the use of the synovium-derived mesenchymal stem cells for the preparation of a pharmaceutical composition for treating bone or cartilage damage.

### BRIEF DESCRIPTION OF THE DRAWINGS

FIG. 1A shows microscopic images showing the results of culturing synovial fragments for 14 days;
FIG. 1B shows microscopic images showing the results of sub-culturing synMSCs for 15 passages, in which Passage 1 represents subculture passage 1 (P1), Passage 5 represents subculture passage 5 (P5), and Passage 15 represents subculture passage 15 (P15);
FIG. 2A shows images showing the results of immunostaining of positive epitopes of synMSCs with a monoclonal antibody;
FIG. 2B shows images showing the results of immunostaining of negative epitopes of synMSCs with a monoclonal antibody;
FIG. 2C shows a graph showing the results of quantitative analysis of fluorescence developed during immunostaining of synMSCs;
FIG. 3A shows fluorescence microscopic images showing the results of performing Oil Red O staining, after inducing differentiation of synMSCs into adipocytes;
FIG. 3B shows a graph showing the results of quantitative analysis of Oil Red O staining levels which were measured in adipocytes differentiated from synMSCs;
FIG. 3C shows fluorescence microscopic images showing the results of performing Alizarin Red S staining, after inducing differentiation of synMSCs into osteocytes;
FIG. 3D shows a graph showing the results of quantitative analysis of Alizarin Red S staining levels which were measured in osteocytes differentiated from synMSCs;
FIG. 3E shows fluorescence microscopic images showing BCIP/NBT color development levels by ALP activity, after inducing differentiation of synMSCs into osteocytes;
FIG. 3F shows a graph showing the results of quantitative analysis of ALP activity which was measured in osteocytes differentiated from synMSCs;
FIG. 4A shows microscopic images showing the results of performing H&E staining and alcian blue staining of control groups, BMSCs and P3 synMSCs;
FIG. 4B shows a graph showing the results of quantitative analysis of H&E staining levels of BMSCs and P3 synMSCs;
FIG. 4C shows a graph showing the results of quantitative analysis of alcian blue staining levels of BMSCs and P3 synMSCs;
FIG. 5A shows a graph showing the results of quantitative analysis of sGAGs (sulfated glycosaminoglycan) levels which were measured in P3 synMSCs (control group), chondrocytes (comparative group) differentiated from P3 synMSCs, and chondrocytes (experimental group) differentiated from P3 synMSCs using BMP-7;
FIG. 5B shows an electrophoresis image showing the results of measuring expression levels of chondrogenic marker proteins (SOX-9, aggrecan, and type 2 collagen) which were expressed in P3 synMSCs (control group), chondrocytes (comparative group) differentiated from P3 synMSCs, and chondrocytes (experimental group) differentiated from P3 synMSCs using BMP-7;
FIG. 5C shows a graph showing the results of quantitative analysis of expression levels of chondrogenic marker proteins (SOX-9, aggrecan, and type 2 collagen) which were expressed in P3 synMSCs (control group), chondrocytes (comparative group) differentiated from P3 synMSCs, and chondrocytes (experimental group) differentiated from P3 synMSCs using BMP-7;
FIG. 6A shows a graph showing the results of analyzing changes in the number of viable cells, after culturing P3 synMSCs using a PLGA scaffold;
FIG. 6B shows a scanning electron microscopic image showing morphological changes over the culture period, when P3 synMSCs were cultured using a PLGA scaffold; and
FIG. 7 shows microscopic images showing the results of performing H&E staining, Safranin-O staining, and type 2 collagen antibody immunostaining of femurs of the rabbit joints, in which a PLGA scaffold, a culture of synMSCs on a PLGA scaffold (PLGA/SynMSC), or a culture of synMSCs on a BMP-7-loaded PLGA scaffold (PLGA/SynMSC/BMP-7) was implanted.

### DETAILED DESCRIPTION OF THE PREFERRED EMBODIMENTS

To achieve the above-described objects, an aspect of the present invention defined in claims 1 to 3 provides a method of preparing synovium-derived mesenchymal stem cells using a synovial tissue and a hydrogel. The method of preparing synovium-derived mesenchymal stem cells includes the steps of (a) encapsulating the synovial tissue in the hydrogel and culturing the same to obtain a culture; and (b) degrading the hydrogel in the obtained culture to recover, from the synovial tissue, the mesenchymal stem cells that migrate to and proliferate in the hydrogel, wherein the synovium-derived mesenchymal stem cells have immunological characteristics of overexpressing CD29, CD44, CD73 and CD90 on the cell surface thereof.

As used herein, the term "synovium", which is also called synovial membrane or synovial stratum, refers to a loose connective tissue that constitutes the inner layer of articular capsules surrounding the joint cavity. It is known that capillary blood vessels are developed in the synovium to actively exchange synovial fluid. The synovium has a different volume depending on the joint, and in some cases, forms wrinkles to surround the fat pad in the joint cavity and fills the cavity of the joint.

In the present invention, the synovium may be understood as a source tissue for isolating mesenchymal stem cells.

As used herein, the term "hydrogel" refers to a gel containing water as a dispersion medium. The hydrogel is mainly formed when fluidity is lost due to cooling or when a hydrophilic polymer having a three-dimensional network structure and a microcrystalline structure contains water to swell. Since a hydrogel containing polymer electrolytes exhibits high water absorption, it has been practically used as a water absorbent polymer in a variety of fields.

In the present invention, the hydrophilic polymer constituting the hydrogel is not particularly limited, as long as it is used in the process of preparing the synovium-derived mesenchymal stem cells, and it may be, for example, collagen, gelatin, chondroitin, hyaluronic acid, alginic acid, MatrigelTM, chitosan, peptide, fibrin, polyglycolic acid (PGA), polylactic acid (PLA), polyethylene glycol (PEG), polyacrylamide, etc., and a mixture thereof.

In the step (a) of the method of preparing synovium-derived mesenchymal stem cells provided in the present invention, a method of encapsulating the synovial tissue in the hydrogel is not particularly limited, but it may be performed by a method of mixing the synovial tissue with the hydrophilic polymer constituting the hydrogel, and then transforming the hydrophilic polymer to the hydrogel; or a method of forming the hydrogel, and then physically injecting the synovial tissue inside the hydrogel, etc.

In the step (a), the culturing of the hydrogel, in which the synovial tissue is encapsulated, may be performed after immersing the hydrogel, in which the synovial tissue is encapsulated, in a common medium which is known in the art to be suitable for culturing stem cells.

The medium is not particularly limited, but for one example, Dulbecco's modified Eagle medium (DMEM) or Keratinocyte serum free medium (Keratinocyte-SFM) may be used, and for another example, D-media (Gibco) may be used.

The medium may further include various kinds of additives, and for example, a neutral buffer (e.g., phosphate and/or high concentration bicarbonate) in isotonic solution, a protein nutrient (e.g., serum such as FBS, serum replacement, albumin, or essential and non-essential amino acids such as glutamine), lipids (fatty acids, cholesterol, an HDL or LDL extract of serum), and other ingredients (e.g., insulin or transferrin, nucleosides or nucleotides, pyruvate, a sugar source such as glucose, selenium in any ionized form or salt, a glucocorticoid such as hydrocortisone, and/or a reducing agent such as β-mercaptoethanol) may be used as the additive. Furthermore, to prevent cells from adhering to each other, an anti-clumping agent may be used as the additive.

Moreover, in the step (b) of the method of preparing synovium-derived mesenchymal stem cells provided in the present invention, the degrading of the hydrogel is not particularly limited, as long as it is able to degrade the hydrogel without affecting the stem cells in the hydrogel. The degrading of the hydrogel may be performed by, for example, a method of using an enzymatic reaction, and for another example, a method of using an enzyme that cleaves binding of the hydrophilic polymer constituting the hydrogel, such as collagenase, gelatinase, urokinase, streptokinase, a tissue plasminogen activator (TPA), plasmin, hyaluronidase, etc.

Meanwhile, the mesenchymal stem cells prepared by the above method of preparing synovium-derived mesenchymal stem cells exhibit immunological characteristics of expressing CD29, CD44, CD73, and CD90 on the cell surface thereof, and characteristics of differentiating into cells selected from the group consisting of adipocytes, osteocytes, chondrocytes, and combinations thereof.

In particular, when the mesenchymal stem cells are differentiated into chondrocytes by treatment with BMP-7, differentiation efficiency into chondrocytes may be remarkably increased.

Further, proliferation of the mesenchymal stem cells may be promoted by using a PLGA scaffold.

Another aspect of the present invention defined in claims 4 to 8 provides a pharmaceutical composition for treating bone or cartilage damage, the pharmaceutical composition including, as an active ingredient, the synovium-derived mesenchymal stem cells prepared by the method of the invention.

As used herein, the term "treatment" means all of the actions by which symptoms of bone or cartilage damage have taken a turn for the better or been modified favorably by the administration of the synovium-derived mesenchymal stem cells, the culture thereof, or the cells differentiated from the mesenchymal stem cells.

According to one exemplary embodiment of the present invention, it was confirmed that when the synovium-derived mesenchymal stem cells were injected into the damaged site of the cartilage and femur of the knee joint, they exhibited a therapeutic effect of regenerating the damaged bone and cartilage, and this therapeutic effect was improved by BMP-7.

In the present invention, the synovium-derived mesenchymal stem cells may exhibit a therapeutic effect on the damaged bone or cartilage site, and this therapeutic effect may be improved when the mesenchymal stem cells are used in the form of being mixed with a support. Specifically, when the synovium-derived mesenchymal stem cells are used after being cultured in the form of being adhered onto the support, the therapeutic effect may be improved. When the support, of which surface is loaded with BMP-7, is used, the therapeutic effect may be further improved.

In the present invention, the support is not particularly limited, as long as it does not impair the therapeutic effect of the synovium-derived mesenchymal stem cells, and the support may be, for example, a PLGA scaffold.

The level of the synovium-derived mesenchymal stem cells included in the pharmaceutical composition provided in the present invention is not particularly limited, and the pharmaceutical composition may include, for example, 1.0×10⁵ cells to 1.0×10⁹ cells per 1 ml, for another example, 1.0×10⁶ cells to 1.0×10⁸ cells per 1 ml, and for still another example, 1.0×10⁷ cells per 1 ml.

The pharmaceutical composition may be used unfrozen, or may be frozen for later use. If the pharmaceutical composition is required to be frozen, a standard cryopreservative (e.g., DMSO, glycerol, Epilife^{®} cell freezing medium (Cascade Biologics)) may be added to population of cells before being frozen.

Furthermore, the pharmaceutical composition may be administered after formulating it into a unit dosage suitable for administering to a patient by common methods in the pharmaceutical field, in which the formulation contains an effective amount for a single dose or for divided doses. For this purpose, a formulation for parenteral administration preferably includes an injection formulation such as an injection ampoule, an infusion formulation such as an infusion bag, and a spray formulation such as aerosol. The injection ampoule may be mixed with an injection solution immediately before administration, and as the injection solution, a saline solution, a glucose, mannitol, or ringer solution, etc. may be used. Furthermore, the infusion bag may be textured with polyvinyl chloride or polyethylene, and may be exemplified by a product of Baxter, Becton Dickinson, Medcep, National Hospital Products, or Terumo.

The pharmaceutical composition may further include one or more pharmaceutically acceptable common inactive carriers, for example, a preservative, an analgesic controller, a solubilizer, a stabilizer, etc. for injection formulation, and a base, an excipient, a lubricant, a preservative, etc. for topical formulation, in addition to the synovium-derived mesenchymal stem cells.

Furthermore, the pharmaceutical composition provided in the present invention may include various components which may assist in the treatment of bone or cartilage damage, may maintain the activity of the synovium-derived mesenchymal stem cells, or may promote the differentiation of the synovium-derived mesenchymal stem cells, in addition to the synovium-derived mesenchymal stem cells. The pharmaceutical composition may further include, for example, an anti-inflammatory agent, a stem cell-mobilizing factor, a growth inducing factor, etc.

The pharmaceutical composition of the present invention may be administered in accordance with any common method in the art, together with other stem cells used for transplantation and other purposes, in the form of a mixture therewith. Direct engraftment or transplantation to the lesion of a patient in need of treatment, or direct transplantation or injection into the peritoneal cavity is preferred, but is not limited thereto. Furthermore, both of a non-surgical administration using a catheter and a surgical administration such as injection or transplantation after incision are possible, but non-surgical administration using a catheter is more preferred. In addition, the composition may also be administered parenterally, for example, intravenous injection, which is one of the common methods for transplantation of stem cells of hematopoietic system, besides direct administration to the lesion.

The daily dosage of the stem cells is not particularly limited, but the stem cells may be administered, for example, in an amount of 1.0×10⁴ to 1.0×10¹⁰ cells/kg (body weight), and for another example, 1.0×10⁵ to 1.0×10⁹ cells/kg (body weight) per day in a single dose or in divided doses. However, it should be understood that the amount of the active ingredient actually administered ought to be determined in light of various relevant factors including a disease to be treated, severity of the disease, a route of administration, and a patient's body weight, age, and sex, etc., and therefore, the above administration dose should not limit the scope of the present invention in any way.

Still another aspect of the present disclosure is a composition for culturing mesenchymal stem cells, the composition including a synovial tissue and a hydrogel, and a kit for culturing the mesenchymal stem cells, the kit including the composition.

As described above, when the synovial tissue is cultured after being encapsulated in the hydrogel, the synovium-derived mesenchymal stem cells may be effectively prepared, and thus the composition including the synovial tissue and the hydrogel may be used as a composition for culturing the mesenchymal stem cells.

Furthermore, the kit for culturing the mesenchymal stem cells may include the composition for culturing the mesenchymal stem cells and various components, such as a solution and a device, needed for culturing the mesenchymal stem cells.

The components may be exemplified by, but are not particularly limited to, a test tube, an appropriate container, a reaction buffer (varies in pH and buffer concentration), a culture container, a culture medium, sterile water, etc.

Still another aspect of the present disclosure is the use of the synovium-derived mesenchymal stem cells for the preparation of the pharmaceutical composition for treating bone or cartilage damage.

The synovium-derived mesenchymal stem cells for the preparation of the pharmaceutical composition may be mixed with an acceptable excipient, diluent, carrier, etc., and may be formulated into a complex preparation with other active agents to have a synergistic effect of the active ingredients.

Those mentioned in the composition, use, and treatment method described herein are the same as long as they do not contradict each other.

Hereinafter, the present invention will be described in more detail with reference to Examples. However, the following Examples are only for illustrating the present invention, and the scope of the present invention is not intended to be limited by the following Examples.

### Example 1: Acquisition of Synovium-Derived Mesenchymal Stem Cells

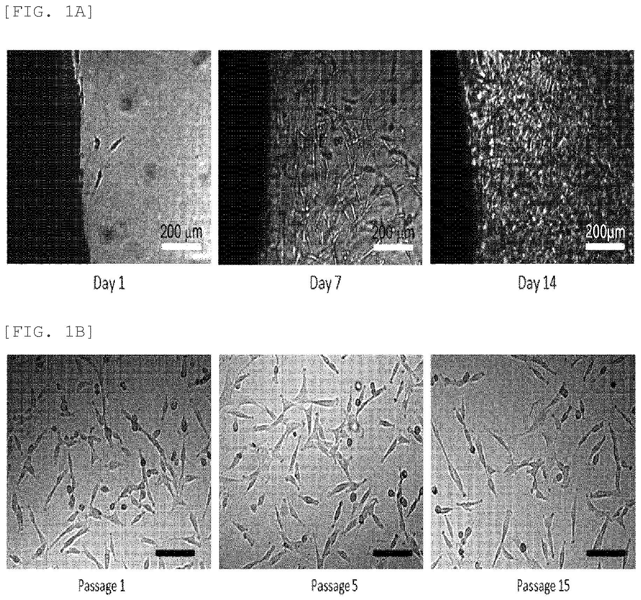

Synovium was obtained from the suprapatellar pouch of both knee joints of a rabbit (New Zealand white rabbit, 6-48 week-old, male). The obtained synovium was finely cut to obtain synovial fragments, which were then washed with PBS. The washed synovial fragments were suspended in a DMEM medium, in which 100 ug/ml of aminomethylbenzoic acid was contained and 1 unit/ml of thrombin was dissolved, and this was mixed with a DMEM medium, in which 40 mmol/L of calcium chloride was contained and 0.5% fibrinogen was dissolved, at 1:1 (v/v) to form a hydrogel. 10 ml of the hydrogel containing about 200 mg of the synovial fragments was put in a 100 mm culture vessel, and incubated in a humidification chamber at 37°C for 2 hours, and then 10 ml of a primary growth medium (90% DMEM-Ham's F12, 10% fetal bovine serum, 10 ng/ml of epidermal growth factor (EGF), 2 ng/ml of basic fibroblast growth factor (bFGF), 10 ng/ml of insulin-like growth factor (IGF), and 10 ug/ml of gentamycin) was added thereto, followed by incubation at 37°C for 14 days in the humidification chamber. After the culturing was completed, the medium was removed, and fibrin forming the hydrogel was degraded by adding a DMEM medium containing 5000 units of urokinase and 30% calf serum, and then centrifuged (150 g, 5 minutes) to obtain synovium-derived mesenchymal stem cells (synMSCs) (FIG. 1A).

FIG. 1A shows microscopic images showing the results of culturing synovial fragments for 14 days.

The obtained synMSCs were washed with PBS twice, and suspended in a secondary growth medium (10% v/v FBS with 10 U/ml antibiotics, 10 ng/ml EGF, and 2 ng/ml bFGF in DMEM/Ham's F-12 (1,1) mixture), and then sub-cultured for 7 days. When the cells reached 80% to 90% confluency, the culturing was terminated. Then, the culture was treated with a 2.5% (w/v) trypsin-EDTA solution to detach the cells, and then centrifuged to obtain Passage 1 (P1) of synMSCs. Thereafter, the same method was repeated to obtain five different series of Passage 15 (P15) of synMSCs (FIG. 1B).

FIG. 1B shows microscopic images showing the results of sub-culturing synMSCs for 15 passages, in which Passage 1 represents subculture passage 1 (P1), Passage 5 represents subculture passage 5 (P5), and Passage 15 represents subculture passage 15 (P15).

As shown in FIG. 1B, it was observed that synovium-derived mesenchymal stem cells (synMSCs) maintained morphological characteristics even after subculturing 15 times.

### Example 2: Immunophenotyping of synMSCs

Among the various passages of synMSCs obtained in Example 1, passage 3 (P3) of synMSCs was subjected to cell surface epitope profiling. At this time, CD29, CD34, CD44, CD45, CD73, and CD90 were targeted as epitopes.

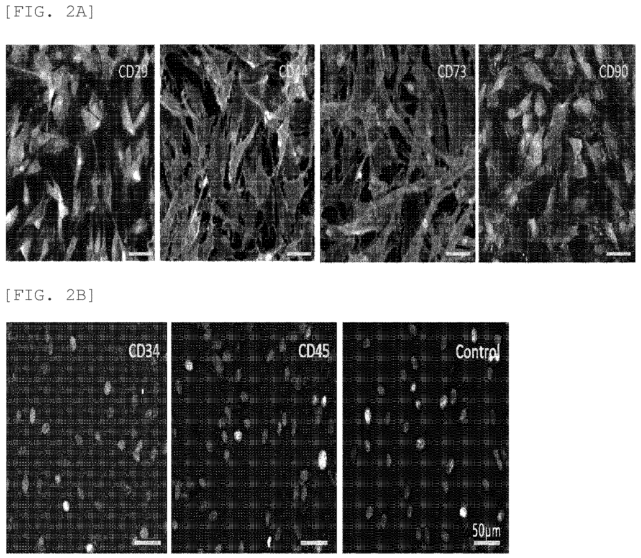

Briefly, 1 × 10⁴ P3 synMSCs per well were dispensed and cultured in a 96-well plate, and after completion of the culturing, PBST containing 1% BSA (PBS containing 0.05% Tween 20) was added to perform blocking. Then, each monoclonal antibody against the epitope (anti-mouse CD29, anti-rabbit CD44, anti-human CD73 PE-Cyanine7, anti-human CD90 PE-CyTM7, anti-CD34 PerCPCy5.5 and anti-rabbit CD45) was added and allowed to react. After the reaction was completed, the cells were washed three times with PBS, 1% BSA and a secondary antibody (goat anti-mouse IgG labeled with Alexa Fluor 488, 1:100) was added, and then further reacted in the dark. After the reaction was completed, the cells were washed three times with PBS again, DAPI (Molecular probes, OR, USA) was added, followed by counterstaining in a dark room at room temperature. After staining was completed, scanning was performed using Operetta^{®}; High Content Imaging System (PerkinElmer, MA, USA), and fluorescence intensity was quantitatively analyzed (FIGS. 2A to 2C). At this time, as a control group, those treated with Alexa Fluor 488 goat anti-mouse IgG (H+L) instead of the monoclonal antibody against the epitope were used.

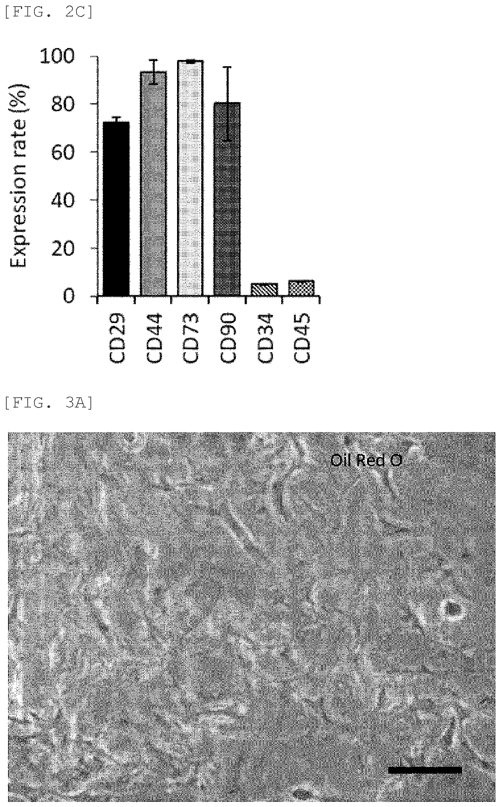

FIG. 2A shows images showing the results of immunostaining of positive epitopes of synMSCs with the monoclonal antibody, FIG. 2B shows images showing the results of immunostaining of negative epitopes of synMSCs with the monoclonal antibody, and FIG. 2C shows a graph showing the results of quantitative analysis of fluorescence developed during immunostaining of synMSCs.

As shown in FIGS. 2A to 2C, it was confirmed that CD29, CD44, CD73, and CD90 were expressed, but CD34 and CD45 were not expressed on the cell surface of synMSCs.

### Example 3: Analysis of Differentiation Potential of synMSCs

### Example 3-1: Differentiation into adipocytes

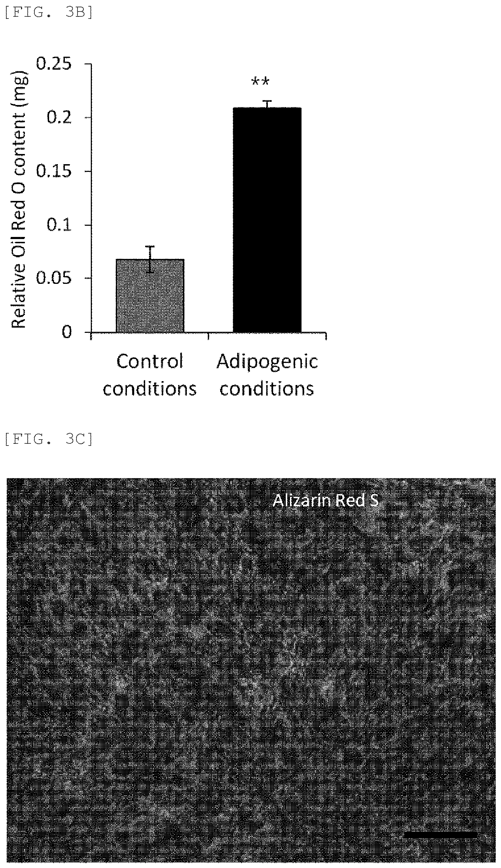

1 × 10⁴ P3 synMSCs per well were dispensed into each well of a 48-well plate containing an adipogenic differentiation induction medium (0.5 mM isobutyl-methylxanthin, 1 µM dexamethasone, 10 µM insulin, 200 µM indomethacin, 1% antibiotic in DMEM), and cultured in a monolayer for 8 days to induce differentiation into adipocytes. As a control group, those cultured in a basic medium (10% FBS in DMEM) were used. After the culturing was completed, cells were fixed by treatment with 4% paraformaldehyde, and Oil Red O staining was performed to detect intracellular lipid droplets which are a feature of adipocytes, and then observed using a fluorescence microscope (FIG. 3A). Thereafter, in order to quantitatively analyze the content of the dye, isopropanol was added to the stained cells to extract the dye, and absorbance at 540 nm was measured (FIG. 3B) .

FIG. 3A shows fluorescence microscopic images showing the results of performing Oil Red O staining, after inducing differentiation of synMSCs into adipocytes, and FIG. 3B shows a graph showing the results of quantitative analysis of Oil Red O staining levels which were measured in adipocytes differentiated from synMSCs.

As shown in FIGS. 3A and 3B, when differentiation of synMSCs into adipocytes was induced, lipid droplets were detected in the cells after 8 days, which was demonstrated by Oil Red O staining.

Therefore, it was found that synMSCs exhibited the differentiation potential into adipocytes.

### Example 3-2: Differentiation into osteocytes

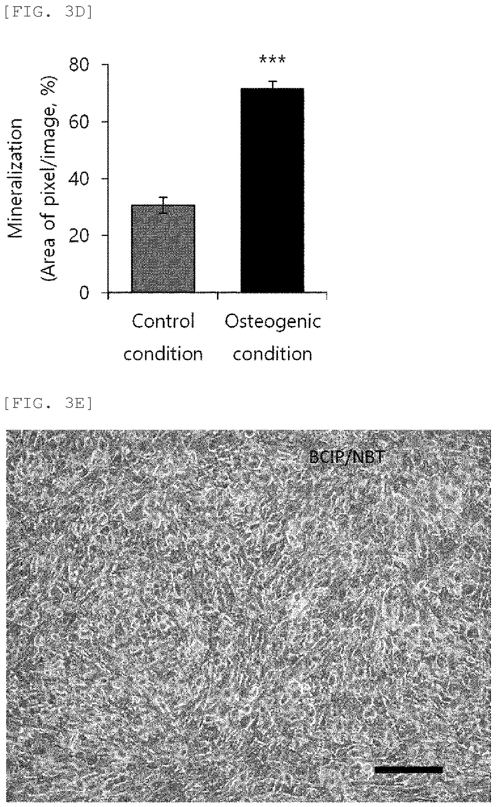

1 × 10⁴ P3 synMSCs per well were dispensed into each well of a 48-well plate containing an osteogenic differentiation induction medium (100 nM dexamethasone, 50 ug/ml ascorbate-2-phosphate, 10 mM β-glycerophosphate, 1% antibiotic suspended in DMEM), and cultured for 14 days to induce differentiation into osteocytes. As a control group, those cultured in a basic medium (10% FBS in DMEM) were used. After the culturing was completed, cells were fixed by treatment with 4% paraformaldehyde, and BCIP/NBT (Sigma Aldrich, MO, USA) which is a substrate of alkaline phosphatase (ALP) was added thereto and allowed to react. After the reaction was completed, Alizarin Red S staining was performed to detect calcium deposits which are a feature of osteocytes, and then observed using a fluorescence microscope, and staining levels thereof were quantitatively analyzed (FIGS. 3C and 3D).

FIG. 3C shows fluorescence microscopic images showing the results of performing Alizarin Red S staining, after inducing differentiation of synMSCs into osteocytes, and FIG. 3D shows a graph showing the results of quantitative analysis of Alizarin Red S staining levels which were measured in osteocytes differentiated from synMSCs.

As shown in FIGS. 3C and 3D, when differentiation of synMSCs into osteocytes was induced, calcium deposits were detected in the cells after 14 days, which was demonstrated by Alizarin Red S staining.

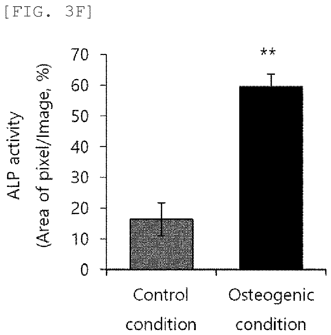

FIG. 3E shows fluorescence microscopic images showing BCIP/NBT color development levels by ALP activity, after inducing differentiation of synMSCs into osteocytes, and FIG. 3F shows a graph showing the results of quantitative analysis of ALP activity which was measured in osteocytes differentiated from synMSCs.

As shown in FIGS. 3E and 3F, it was confirmed that when differentiation of synMSCs into osteocytes was induced, the activity of ALP, which is known as a bone differentiation marker, was increased by about 3 times after 14 days.

Therefore, it was found that synMSCs exhibited the differentiation potential into osteocytes.

### Example 3-3: Differentiation into chondrocytes

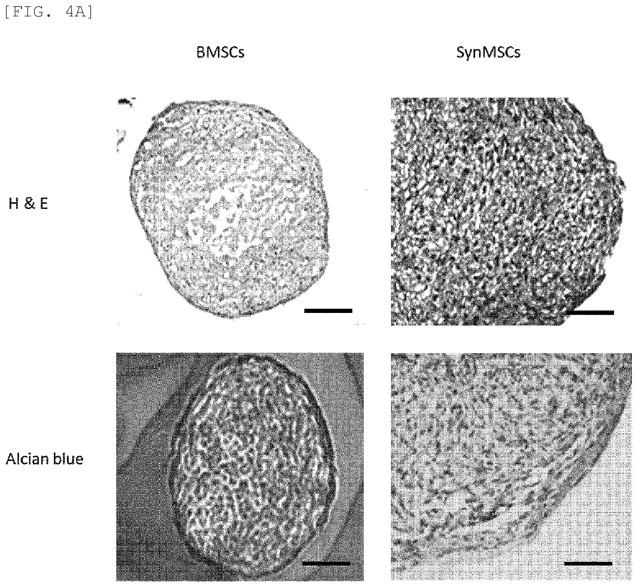

1 × 10⁶ P3 synMSCs per well were dispensed into each well of a poly-D-lysine-coated 6-well culture plate containing a chondrogenic differentiation induction medium (1% calf serum, 1× ITS, 0.1 mM dexamethasone, 50 ug/ml ascorbate-2-phosphate suspended in DMEM), and cultured for 2 weeks to induce differentiation into chondrocytes. On the second day of culture, spheroids were formed from P3 synMSCs, and the medium was replaced every two days. After the culturing was completed, spheroids as culture products were obtained, and fixed by treatment with 4% paraformaldehyde for 2 hours. Subsequently, centrifugation (300 g, 5 min) was performed to obtain spheroids, which were then washed with PBS three times, and then the spheroids were dehydrated by sequentially treating with increasing concentrations of ethanol solutions from 50% to 100%. Then, the spheroids were embedded in paraffin, and 4 µm-thick sections were obtained, followed by H&E staining and alcian blue staining. Nuclear regions were counterstained with nuclear fast red (FIGS. 4A to 4C). At this time, as a control group, a product resulting from inducing differentiation of bone marrow-derived stem cells (BMSCs) under the same conditions was used.

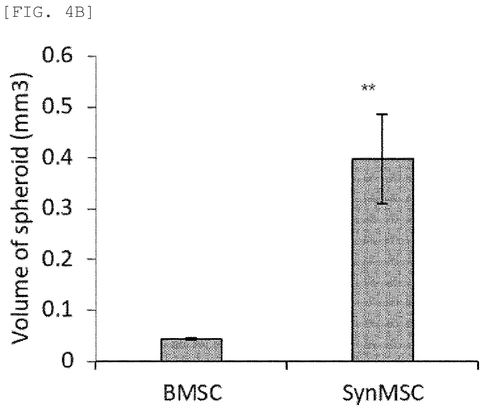

FIG. 4A shows microscopic images showing the results of performing H&E staining and alcian blue staining of control groups, BMSCs and P3 synMSCs, FIG. 4B shows a graph showing the results of quantitative analysis of H&E staining levels of BMSCs and P3 synMSCs, and FIG. 4C shows a graph showing the results of quantitative analysis of alcian blue staining levels of BMSCs and P3 synMSCs.

As shown in FIGS. 4A to 4C, it was confirmed that when differentiation into chondrocytes was induced under the same conditions, the level of chondrocytes differentiated from P3 synMSCs was significantly higher than that of chondrocytes differentiated from the control group BMSCs. In particular, the result of H&E staining showed that chondrocytes differentiated from P3 synMSCs showed about 9 times higher level than chondrocytes differentiated from BMSCs, and the result of alcian blue staining showed that chondrocytes differentiated from P3 synMSCs showed about 13 times higher level than chondrocytes differentiated from BMSCs.

Therefore, it was found that synMSCs exhibited the differentiation potential into chondrocytes.

### Example 3-4: Effect of BMP-7 on differentiation into chondrocytes

### Example 3-4-1: Induction of differentiation of synMSCs into chondrocytes using BMP-7

The differentiation of chondrocytes from P3 synMSCs was induced in the same manner as in Example 3-3, except that a chondrogenic differentiation induction medium supplemented with BMP-7 (50 ng/ml) was used. At this time, a culture, obtained by using a basal medium (10% FBS in DMEM) instead of the chondrogenic differentiation induction medium, was used as a control group, and a culture, obtained by inducing the differentiation of chondrocytes from P3 synMSCs in the same manner as in Example 3-3, was used as a comparative group.

### Example 3-4-2: Analysis of sulfated glycosaminoglycan (sGAGs) content

Sulfated glycosaminoglycans (SGAGs) were quantitatively analyzed for the control group, comparative group, and experimental group obtained in Example 3-4-1.

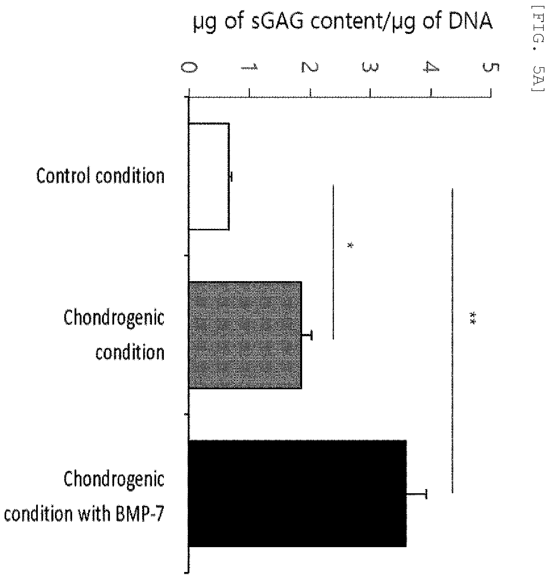

Briefly, blyscan dye was added to the cell extract of each sample, reacted for 30 minutes under shaking conditions, and then centrifuged (12,000 rpm, 10 minutes) to obtain a precipitate. A dissociation reagent was added to the obtained precipitate to obtain a suspension, and absorbance at 656 nm was measured (FIG. 5A) .

FIG. 5A shows a graph showing the results of quantitative analysis of sGAGs (sulfated glycosaminoglycan) levels which were measured in P3 synMSCs (control group), chondrocytes (comparative group) differentiated from P3 synMSCs, and chondrocytes (experimental group) differentiated from P3 synMSCs using BMP-7.

As shown in FIG. 5A, it was confirmed that a significantly high level of sGAGs was detected in chondrocytes (experimental group) differentiated from P3 synMSCs using BMP-7, as compared with chondrocytes (comparative group) differentiated from P3 synMSCs.

### Example 3-4-3: Analysis of expression levels of chondrogenic markers

The expression levels of chondrogenic marker proteins (SOX-9, aggrecan, and type 2 collagen) were analyzed using RT-PCR for the control group, comparative group, and experimental group obtained in Example 3-4-1.

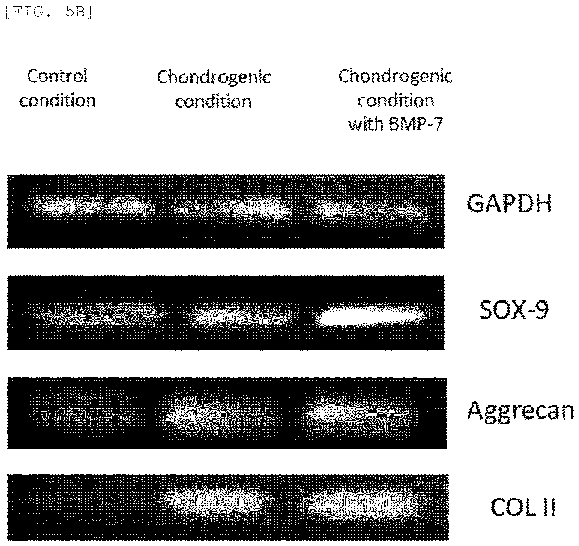

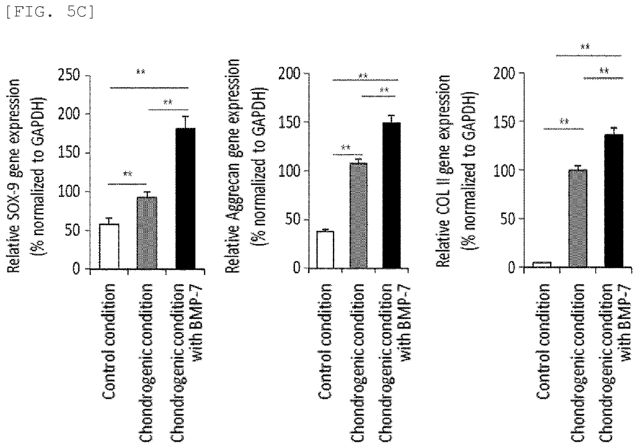

Briefly, cells of each sample were disrupted and total RNA was extracted using Trizol reagent (Invitrogen, CA), respectively. Each extracted total RNA was applied to a TOPscript^{™} One-step RT PCR kit (Enzynomics, Daejeon, Korea) to synthesize each cDNA. PCR was performed using each of the synthesized cDNAs as a template and the following primers to obtain respective amplification products, and levels thereof were quantitatively analyzed (FIGS. 5B and 5C). At this time, GAPDH was used as an internal control group.
SOX-9 F: 5'-CCCGATCTGAAGAAGGAGAGC-3'(SEQ ID NO: 1)
SOX-9 R: 5'-GTTCTTCACCGACTTCCTCCG-3'(SEQ ID NO: 2)
aggrecan F: 5'-TGAGGAGGGCTGGAACAAGTACC-3'(SEQ ID NO: 3)
aggrecan R: 5'-GGAGGTGGTAATTGCAGGGAACA-3'(SEQ ID NO: 4)
T2 collagen F: 5'-TTCAGCTATGGAGATGACAATC-3'(SEQ ID NO: 5)
T2 collagen R: 5'-AGAGTCCTAGAGTGACTGAG-3'(SEQ ID NO: 6)
GAPDH F: 5'-ATTGTTGCCATCAATGACCC-3'(SEQ ID NO: 7)
GAPDH R: 5'-AGTAGAGGCAGGGATGATGTT-3'(SEQ ID NO: 8)

FIG. 5B shows an electrophoresis image showing the results of measuring expression levels of chondrogenic marker proteins (SOX-9, aggrecan, and type 2 collagen) which were expressed in P3 synMSCs (control group), chondrocytes (comparative group) differentiated from P3 synMSCs, and chondrocytes (experimental group) differentiated from P3 synMSCs using BMP-7, and FIG. 5C shows a graph showing the results of quantitative analysis of expression levels of chondrogenic marker proteins (SOX-9, aggrecan, and type 2 collagen) which were expressed in P3 synMSCs (control group), chondrocytes (comparative group) differentiated from P3 synMSCs, and chondrocytes (experimental group) differentiated from P3 synMSCs using BMP-7.

As shown in FIGS. 5B and 5C, it was confirmed that significantly high levels of chondrogenic marker proteins (SOX-9, aggrecan, and type 2 collagen) were detected in chondrocytes (experimental group) differentiated from P3 synMSCs using BMP-7, as compared with chondrocytes (comparative group) differentiated from P3 synMSCs.

### Example 4: Effect of PLGA scaffold on cell proliferation

### Example 4-1: Preparation of PLGA scaffold

A 20% (w/v) polymer solution obtained by dissolving PLGA in dichloromethane (DCM) was placed in a 10 ml syringe with a 17-gauge needle, and wet-spun, and then PLGA fibers were obtained using a roller. The obtained PLGA fibers were dried for 48 hours, and then vacuum-dried at -70°C for 3 days to remove the residual solvent.

A three-dimensional PLGA scaffold was formed using the obtained PLGA fiber, immersed in 1.0 M PBS (pH 7.4), and stirred at 37°C at 60 rpm to remove acidic by-products resulting from degradation of PLGA. The PLGA scaffold, from which the acidic by-products were removed, was washed with PBS three times, and vacuum-dried for 48 hours to prepare a three-dimensional scaffold for P3 synMSC culture.

### Example 4-2: Effect of PLGA scaffold

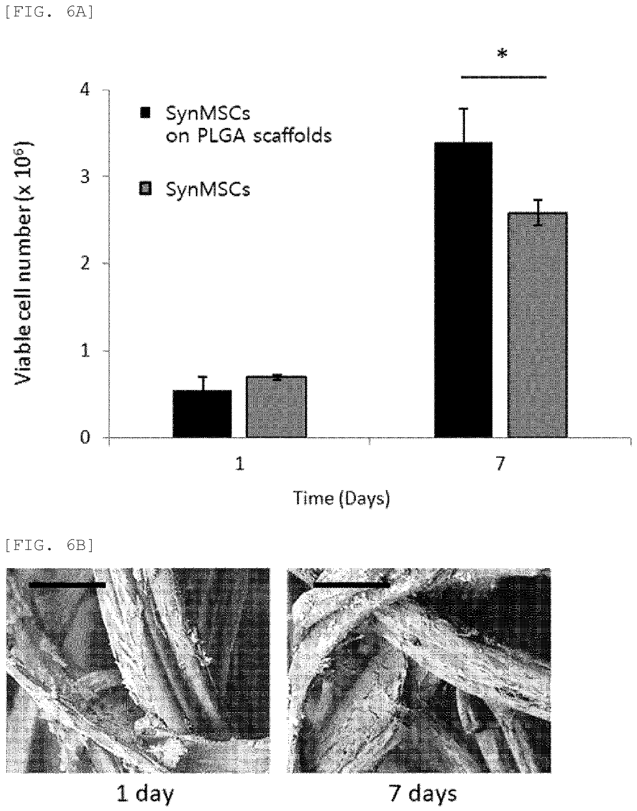

The PLGA scaffold prepared in Example 4-1 was wetted with ethanol, and 1 × 10⁶ P3 synMSCs per the scaffold were inoculated, washed with PBS twice, and cultured for 7 days in a secondary growth medium. After the culturing was completed, the number of viable cells was analyzed using a colorimetric CCK-8 assay (Dojindo Molecular Technologies Inc., MD, USA), and the morphology was analyzed with a scanning electron microscope (SEM) (FIGS. 6A and 6B). At this time, P3 synMSCs which were cultured in a 24-well plate under the same conditions were used as a control group.

FIG. 6A shows a graph showing the results of analyzing changes in the number of viable cells, after culturing P3 synMSCs using the PLGA scaffold, and FIG. 6B shows a scanning electron microscopic image showing morphological changes over the culture period, when P3 synMSCs were cultured using the PLGA scaffold.

As shown in FIG. 6A and 6B, when the PLGA scaffold rather than the 24-well plate was used, the culture efficiency of P3 synMSC was confirmed to increase. This is because the adhesion rate of P3 synMSCs to the PLGA scaffold was increased over time, and as a result, the proliferation rate was increased.

### Example 4-3: Preparation of BMP-7-loaded PLGA scaffold

Recombinant human BMP-7 protein (3 ug) was dissolved in 10 µL of PBS, and then emulsified with 0.2% (w/v) PLGA in acetone/ethanol (9:1) solvent to obtain a water-in-oil suspension of BMP-7 (1:100 w/o ratio), which was placed in a 10 ml syringe with a 17-gauge needle, and spun onto PLGA fibers by electrospraying (10 kV voltage, 0.033 ml/min flow rate), thereby preparing a PLGA scaffold, in which encapsulated BMP-7 was loaded on the PLGA fibers.

### Example 5: Treatment of cartilage damage using synMSCs

The PLGA scaffold prepared in Example 4-1, the culture of synMSCs on the PLGA scaffold (PLGA/SynMSC), prepared in Example 4-2, and the culture of synMSCs on the BMP-7-loaded PLGA scaffold (PLGA/SynMSC/BMP-7), prepared in Example 4-3, were prepared, respectively, and used to prepare each implant having a thickness of 1 mm and a length of 10 mm.

Meanwhile, xylazine (5 mg/kg) was intramuscularly injected into 4-month old male New Zealand white rabbits with an average weight of 3.2 kg to induce general anesthesia, and the knee joint was incised to expose the patella. About 1 mm thick cartilage was removed from the exposed patella, and three osteochondral defects (diameter of 2 mm and depth of 3 mm) were created in in the groove of the femur using a dental drill. Each implant prepared previously was implanted into the created defect site to terminate the operation, tramadol (5 mg/kg) and oxytetracycline (20 mg/kg) were injected, and then raised under normal conditions.

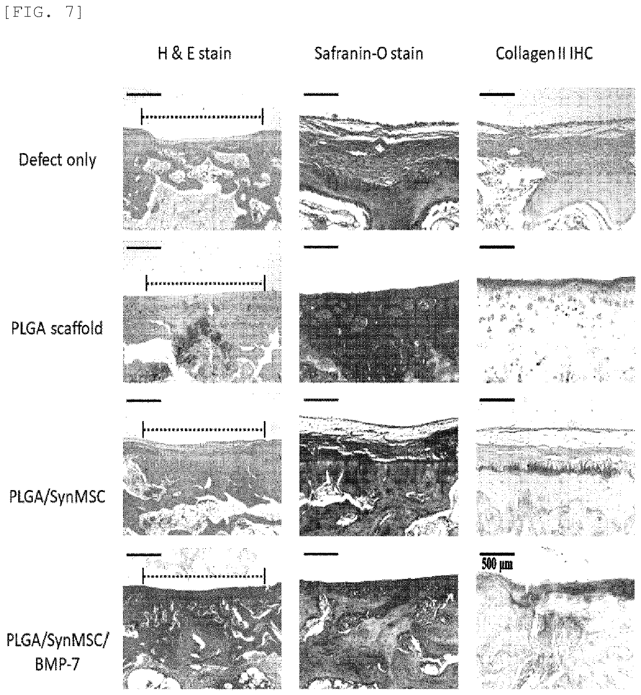

After 6 weeks, each rabbit was sacrificed, and the femur was removed from the knee thereof, and then fixed for 5 days using a 10% neutral buffered formalin (pH 7.4, BBC Biochemical, Mount Vernon, USA, WA) solution. The fixed femur was treated with a 0.5% EDTA solution to remove calcium deposits, and then embedded in paraffin. The embedded femur was finely cut to obtain 4 um-thick tissue sections, which were subjected to H&E staining and Safranin-O staining (FIG. 7).

In addition, the tissue sections were immersed in 3% hydrogen peroxide in methanol for 30 minutes to inhibit the endogenous peroxidase activity, and then proteinase K (Sigma-Aldrich, MO, USA) was added thereto and reacted at 37°C for 10 minutes. After the reaction was completed, the tissue sections were stained with an antibody against type 2 collagen (Calbiochem, CA, USA, dilution 1:100), and immunostaining was performed using a Vectastain Elite ABC-Peroxidase kit (Vector Laboratories Inc., CA, USA). Antibody reaction was color-developed using Vector SG (Vector Laboratories Inc., CA, USA), and counterstaining was performed using nuclear fast solutions (Vector Laboratories Inc., CA, USA) (FIG. 7).

FIG. 7 shows microscopic images showing the results of performing H&E staining, Safranin-O staining, and type 2 collagen antibody immunostaining of femurs of the rabbit joints, in which the PLGA scaffold, the culture of synMSCs on the PLGA scaffold (PLGA/SynMSC), or the culture of synMSCs on the BMP-7-loaded PLGA scaffold (PLGA/SynMSC/BMP-7) was implanted.

As shown in FIG. 7, in the control group, in which the damaged site was not treated, the layer of cartilage was not regenerated, and the site was simply covered with the fibrous tissue, and Safranin-O staining did not occur. However, at the site where the PLGA scaffold implant was implanted, a cartilage layer was formed, but a thin cartilage layer containing a small number of chondrocytes was formed, and proteoglycan was also formed in a small amount. It was also confirmed that, at the site where the implant of synMSCs cultured on the PLGA scaffold was implanted, the cell level was increased and cartilage was regenerated on the surface. Finally, it was confirmed that, at the site where the implant of synMSCs cultured on the BMP-7-loaded PLGA scaffold was implanted, a thick cartilage layer was regenerated and a large amount of type 2 collagen was present.

Accordingly, it was found that synMSCs exhibited a therapeutic effect of regenerating damaged bone and cartilage, and this therapeutic effect was improved by BMP-7.

Based on the above description, it will be understood by those skilled in the art that the present disclosure may be implemented in a different specific form or essential characteristics thereof. In this regard, it should be understood that the above embodiment is not limitative, but illustrative in all aspects. The scope of the disclosure is defined by the appended claims rather than by the description preceding them, and therefore all changes and modifications that fall within metes and bounds of the claims, or equivalents of such metes and bounds are therefore intended to be embraced by the claims.

### Effect of the invention

When a preparation method of the present invention is used, stem cells may be effectively extracted and obtained from synovium, and the obtained synovium-derived mesenchymal stem cells may effectively treat bone or cartilage damage, and therefore, it will be able to greatly contribute to the development of a method of treating bone or cartilage damage.

## Claims

1. A method of preparing synovium-derived mesenchymal stem cells, the method comprising the steps of:
(a) encapsulating a synovial tissue in a hydrogel and culturing the same to obtain a culture; and
(b) degrading the hydrogel in the obtained culture to recover, from the synovial tissue, mesenchymal stem cells that migrate to and proliferate in the hydrogel,
wherein the synovium-derived mesenchymal stem cells have immunological characteristics of expressing CD29, CD44, CD73, and CD90 on the cell surface thereof.

2. The method of claim 1, wherein the hydrogel consists of a material selected from the group consisting of collagen, gelatin, chondroitin, hyaluronic acid, alginic acid, MatrigelTM, chitosan, peptide, fibrin, polyglycolic acid (PGA), polylactic acid (PLA), polyethylene glycol (PEG), polyacrylamide, and combinations thereof.

3. The method of claim 1, wherein the degrading of the hydrogel is performed by treatment with an enzyme selected from the group consisting of collagenase, gelatinase, urokinase, streptokinase, a tissue plasminogen activator (TPA), plasmin, hyaluronidase, and combinations thereof.

4. A pharmaceutical composition for treating bone or cartilage damage, the pharmaceutical composition comprising, as an active ingredient, the synovium-derived mesenchymal stem cells prepared by the method of any one of claims 1 to 3, a culture thereof,
wherein the mesenchymal stem cells are in a form where the cells are cultured on a three-dimensional PLGA scaffold support and adhered thereto; and
the PLGA scaffold support is in a form where BMP-7 are spun onto PLGA fibers and thereby encapsulated BMP-7 is bound to the PLGA fibers.

5. The pharmaceutical composition of claim 4, wherein the synovium-derived mesenchymal stem cells have enhanced ability to regenerate cartilage by the addition of BMP-7.

6. The pharmaceutical composition of claim 4, wherein the synovium-derived mesenchymal stem cells have increased GAG synthesis.

7. The pharmaceutical composition of claim 4, wherein the synovium-derived mesenchymal stem cells have increased expression levels of SOX-9, aggrecan, and type 2 collagen.

8. A pharmaceutical composition for treating bone or cartilage damage, the pharmaceutical composition comprising, as an active ingredient, the synovium-derived mesenchymal stem cells prepared by the method of any one of claims 1 to 3, a culture thereof,
wherein the synovium-derived mesenchymal stem cells have immunological characteristics of expressing CD29, CD44, CD73, and CD90 on the cell surface thereof.

## Patentansprüche

1. Verfahren zur Herstellung von aus Synovium gewonnenen mesenchymalen Stammzellen, wobei das Verfahren die Schritte umfasst:
(a) Einkapseln eines Synovialgewebes in ein Hydrogel und Kultivieren desselben, um eine Kultur zu erhalten; und
(b) Abbauen des Hydrogels in der erhaltenen Kultur, um aus dem Synovialgewebe mesenchymale Stammzellen zu gewinnen, die in das Hydrogel migrieren und darin proliferieren,
wobei die von aus Synovium gewonnenen mesenchymalen Stammzellen die immunologischen Eigenschaften aufweisen, CD29, CD44, CD73 und CD90 auf der Zelloberfläche davon zu exprimieren.

2. Verfahren nach Anspruch 1, wobei das Hydrogel aus einem Material besteht, das ausgewählt ist aus der Gruppe bestehend aus Kollagen, Gelatine, Chondroitin, Hyaluronsäure, Alginsäure, MatrigelTM, Chitosan, Peptid, Fibrin, Polyglykolsäure (PGA), Polymilchsäure (PLA), Polyethylenglykol (PEG), Polyacrylamid und Kombinationen davon.

3. Verfahren nach Anspruch 1, wobei der Abbau des Hydrogels durch Behandlung mit einem Enzym durchgeführt wird, das ausgewählt ist aus der Gruppe, bestehend aus Kollagenase, Gelatinase, Urokinase, Streptokinase, einem Gewebeplasminogenaktivator (TPA), Plasmin, Hyaluronidase und Kombinationen davon.

4. Pharmazeutische Zusammensetzung zur Behandlung von Knochen- oder Knorpelschäden, wobei die pharmazeutische Zusammensetzung als einen Wirkstoff die von aus Synovium gewonnenen mesenchymalen Stammzellen, die durch das Verfahren nach einem beliebigen der Ansprüche 1 bis 3 hergestellt wurden, eine Kultur davon, umfasst,
wobei die mesenchymalen Stammzellen in einer Form vorliegen, in der die Zellen auf einem dreidimensionalen PLGA-Gerüstträger kultiviert werden und daran haften; und
der PLGA-Gerüstträger in einer Form vorliegt, in der BMP-7 auf PLGA-Fasern gesponnen und dadurch eingekapselt wird, wobei eingekapseltes BMP-7 an die PLGA-Fasern gebunden ist.

5. Pharmazeutische Zusammensetzung nach Anspruch 4, wobei die aus Synovium gewonnenen mesenchymalen Stammzellen durch die Zugabe von BMP-7 verbesserte Fähigkeit zur Knorpelregeneration aufweisen.

6. Pharmazeutische Zusammensetzung nach Anspruch 4, wobei die aus Synovium gewonnenen mesenchymalen Stammzellen erhöhte GAG-Synthese aufweisen.

7. Pharmazeutische Zusammensetzung nach Anspruch 4, wobei die aus Synovium gewonnenen mesenchymalen Stammzellen erhöhte Expressionsniveaus von SOX-9, Aggrecan und Typ-2-Kollagen aufweisen.

8. Pharmazeutische Zusammensetzung zur Behandlung von Knochen- oder Knorpelschäden, wobei die pharmazeutische Zusammensetzung als einen Wirkstoff die aus Synovium gewonnenen mesenchymalen Stammzellen, die durch das Verfahren nach einem beliebigen der Ansprüche 1 bis 3 hergestellt wurden, eine Kultur davon, umfasst,
wobei die aus Synovium gewonnenen mesenchymalen Stammzellen immunologische Eigenschaften aufweisen, CD29, CD44, CD73 und CD90 auf der Zelloberfläche davon zu exprimieren.

## Revendications

1. Procédé de préparation de cellules souches mésenchymateuses dérivées de synovie, le procédé comprenant les étapes de :
(a) encapsulation d'un tissu synovial dans un hydrogel et culture de ce dernier pour obtenir une culture ; et
(b) dégradation de l'hydrogel dans la culture obtenue pour récupérer, à partir du tissu synovial, des cellules souches mésenchymateuses qui migrent vers l'hydrogel et y prolifèrent,
dans lequel les cellules souches mésenchymateuses dérivées de synovie ont des caractéristiques immunologiques d'expression de CD29, CD44, CD73 et CD90 sur leur surface cellulaire.

2. Procédé selon la revendication 1, dans lequel l'hydrogel est constitué d'un matériau choisi dans le groupe constitué par le collagène, la gélatine, la chondroïtine, l'acide hyaluronique, l'acide alginique, le MatrigelTM, le chitosane, les peptides, la fibrine, l'acide polyglycolique (PGA), l'acide polylactique (PLA), le polyéthylène glycol (PEG), le polyacrylamide et leurs combinaisons.

3. Procédé selon la revendication 1, dans lequel la dégradation de l'hydrogel est réalisée par traitement avec une enzyme choisie dans le groupe constitué par la collagénase, la gélatinase, l'urokinase, la streptokinase, un activateur tissulaire du plasminogène (TPA), la plasmine, la hyaluronidase et leurs combinaisons.

4. Composition pharmaceutique pour le traitement de lésions osseuses ou cartilagineuses, la composition pharmaceutique comprenant, en tant que principe actif, les cellules souches mésenchymateuses dérivées de synovie préparées par le procédé selon l'une quelconque des revendications 1 à 3, une culture de celles-ci,
dans laquelle les cellules souches mésenchymateuses sont sous une forme dans laquelle les cellules sont cultivées sur un support échafaudage de PLGA tridimensionnel et adhèrent à ce dernier ; et
le support échafaudage de PLGA est sous une forme dans laquelle de la BMP-7 est filée sur des fibres de PLGA et ainsi la BMP-7 encapsulée est liée aux fibres de PLGA.

5. Composition pharmaceutique selon la revendication 4, dans laquelle les cellules souches mésenchymateuses dérivées de synovie ont une capacité améliorée à régénérer le cartilage par l'ajout de BMP-7.

6. Composition pharmaceutique selon la revendication 4, dans laquelle les cellules souches mésenchymateuses dérivées de synovie présentent une synthèse accrue de GAG.

7. Composition pharmaceutique selon la revendication 4, dans laquelle les cellules souches mésenchymateuses dérivées de synovie présentent des niveaux d'expression accrus de SOX-9, d'aggrécane et de collagène de type 2.

8. Composition pharmaceutique pour le traitement de lésions osseuses ou cartilagineuses, la composition pharmaceutique comprenant, en tant que principe actif, les cellules souches mésenchymateuses dérivées de synovie préparées par le procédé selon l'une quelconque des revendications 1 à 3, une culture de celles-ci,
dans laquelle les cellules souches mésenchymateuses dérivées de synovie présentent des caractéristiques immunologiques d'expression de CD29, CD44, CD73 et CD90 sur leur surface cellulaire.
